# EUROPEAN PATENT SPECIFICATION

(11) **EP 0 511 805 B1**
(45) Date of publication and mention of the grant of the patent: **02.07.1997**
(21) Application number: 92303767.5
(22) Date of filing: 27.04.1992
(51) Int. Cl.: A61B 17/36, G02B 27/00, G02B 13/00, G02B 17/06, B23K 26/06, G02B 6/42

(54) **Coupler for a laser delivery system**
Koppler für ein Laserstrahlzuführsystem
Coupleur pour un système de transmission d'un faisceau laser

(30) Priority: 01.05.1991 US 694245; 29.07.1991 US 737395
(43) Date of publication of application: 04.11.1992
(73) Proprietor: COHERENT, INC., Santa Clara, CA 95056 (US)
(72) Inventor: Arnett, Michael, Palo Alto, California 94301 (US); Trost, David, San Francisco, California 94109 (US); Koop, Dale, Sunnyvale, California 94087 (US)
(74) Representative: Jackson, David Spence

(56) References cited:
- EP-A- 0 196 519
- EP-A- 0 351 240
- WO-A-89/06518
- DE-A- 2 059 821
- DE-A- 2 803 898
- DE-A- 2 810 879
- US-A- 3 982 541
- US-A- 4 144 888
- US-A- 5 000 553

## Description

This invention relates to a coupler for coupling a medical laser beam delivery system to an elongate instrument, the delivery system including an arm for delivering a laser beam, and said instrument having an axially extending bore through which the laser beam is transmitted, the coupler having a body with one end adapted to be connected to the said arm and the other end including an adapter flange having an axially projecting alignment tube receivable within the bore of the instrument.

A coupler is disclosed particularly suited for use in surgical laser beam delivery systems. The coupler is configured to improve the efficiency of the transmission of the laser beam through the delivery system.

The use of lasers in various medical procedures is becoming quite common. The type of device used to deliver the laser energy from the laser to the treatment site is primarily dependent upon the wavelength of light generated. For example, 1.06 micron radiation generated by a Nd:YAG laser is typically delivered by an optical fibre formed from a silica compound. In contrast, 10.6 micron radiation generated by a CO₂ laser is typically delivered through an articulated arm assembly since presently existing optical fibres will not efficiently transmit such longer wavelength radiation.

An articulated arm consists of a number of hollow segments connected by rotatable joints. Mirrors are located in each of the joints to redirect the laser beam down the next segment of the arm. One end of the arm is connected to the output of the laser while the other end carries a delivery element. An example of an articulated arm is described in US-A-5 000 553.

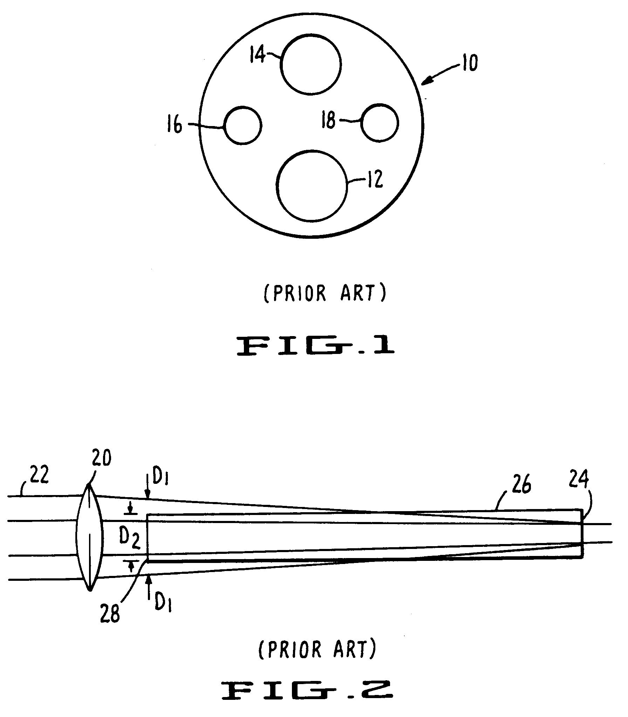

A delivery element is selected based on the type of medical procedure which is to be performed.
A common delivery element is an endoscope 10 which is illustrated in cross section in Figure 1. The endoscope in Figure 1 is of the type that might be used in a laparoscopy and is often referred to as a laparoscope.

As seen in Figure 1, the laparoscope body will typically have a number of axially extending bores 12, 14, 16 and 18. The laser beam may be directed through bore 12. Bore 14 may be used for optical feedback so the surgeon can view the tissue site. The two smaller bores 16 and 18 can be used to deliver illumination via fiber optic bundles to the treatment site.

In the prior art, a coupler is used to join the end of the articulated arm to the entrance of the endoscope. In the present commercial environment, a number of medical device companies manufacture and sell endoscopes each of which have slightly different entrance end configurations. For each different endoscope design, there exists a coupler with a complimentary mating design.

While there exists a wide variety of prior art coupler designs, all of the existing designs have a few common features. For example, the coupler is typically provided with a conically shaped male mating end which is received in a conically shaped female mating configuration formed at the entrance of the endoscope. The mating conical configurations allow the two pieces to be adjusted when mounted. The alignment of the system relies heavily on the accuracy of the mating cones.

Unfortunately, the latter mounting approach has been less than satisfactory. More particularly, the prior approach required the doctor to adjust and test the alignment of the beam each time a new endoscope was attached. Since a new sterile endoscope is used for each new surgical procedure, the doctor must repeatedly adjust the alignment of the system. More significantly, even if the doctor is able to initially align the beam, the continuous movement of the articulated arm during the surgical procedure often results in the alignment being lost. If the alignment is lost, the percentage of the beam power transmitted down the endoscope is adversely affected. Moreover, misalignment of the components affects beam shape and size. Accordingly, it would be desirable to provide an improved coupler which could provide accurate and stable alignment of the mating components.

A coupler of the kind defined hereinbefore at the beginning and described in EP-A-0 196 519 couples a hollow needle for use in transmyocardial revascularization to an articulated arm through which a laser beam from a carbon dioxide laser is delivered. A series of mirrors in the arm directs the beam to a final section or handpiece of the arm. The handpiece has a tubular body in which a focusing lens is mounted that focuses the laser beam at a fixed focal point at the tip of the hollow needle which is coupled to the handpiece by the coupler. The end of the needle that is mounted in the coupler is enlarged and externally threaded to thread into the coupler which is adjustably positioned within a cylindrical holder fixed in the end of the handpiece by an annular cap. The coupler has a hollow cylindrical body fixed within the cylindrical holder by a pair of opposed set screws and internally threaded to engage the enlarged end of the needle. The hollow cylindrical body also has an internal flange from which a tubular extension projects into the bore of the needle where it fits at the enlarged end of the needle.

Another common feature of prior art couplers was the inclusion of a single, positive focusing lens. The lens was used to focus the beam entering the coupler from the articulated arm into the endoscope. The power of the lens was designed to bring the beam to a focus just beyond the distal end of the endoscope.

The latter design has been used quite successfully with endoscopes wherein the diameter of the bore was quite large. When the bore diameter is large, a high percentage of the beam can be injected down the endoscope. Recently, there has been a trend to develop endoscopes with smaller outer diameters and associated smaller axial bores. As can be appreciated, doctors prefer smaller diameter endoscopes because they are easier to handle and the associated incisions can be made smaller. There has also been a trend to develop endoscopes wherein the diameter of the bore used to transmit the laser beam is reduced while increasing the diameter of the bore of the viewing channel to improve visibility at the treatment side.

When the diameter of the axial bore for transmitting laser radiation is reduced for either of the above discussed reasons, problems have arisen with the coupling of the laser beam into the endoscope. Figure 2 illustrates the problem. As can be seen, when the focal power of the lens 20 is selected to bring the focus of the beam 22 near the end 24 of the endoscope 26, the diameter D₁ of the beam at the entrance 28 will be significantly larger than the diameter D₂ of the endoscope bore. This disparity results in severe clipping of the beam at the entrance reducing the power injected into the endoscope.

A further complication arises as the articulated arm is moved during the procedure. As the arm moves, the level of clipping is varied and the delivered power continuously changes. In addition, the beam is distorted as it reflects off the bore. This distortion causes an irregular shape burn that constantly changes with arm movement. Accordingly, it would be desirable to provide an improved coupler which overcame these problems.

WO 89/06518 describes the use in the final portion of an articulated arm of a dental laser system of a condensing system comprising two plano-convex lenses that reduce the diameter of the laser beam to 2 millimetres.

Accordingly, it is an object of the subject invention to provide a new and improved coupler for use with medical laser delivery systems.

It is another object of the subject invention to provide a coupler configured to improve the alignment with the endoscope.

It is a further object of the subject invention to provide a coupler having an axially projecting alignment tube, receivable within the bore of the endoscope for improving alignment.

According to the invention, a coupler of the kind defined hereinbefore at the beginning is characterised in that the adapter flange is configured or adapted to mate with the entrance to the instrument, and in that the alignment tube includes a radially projecting annulus having an outer diameter substantially corresponding to the inner diameter of the bore of the instrument, and in that the annulus includes at least one axial slot to permit some compression of the annulus.

In a preferred embodiment of the invention, the coupler has a telescope focusing system for reducing the clipping of the beam entering an endoscope, and has an axially projecting tube which is receivable in the bore of the endoscope. The tube allows the position of the coupler to be registered with respect to the inner surface of the bore. By this arrangement, there is no need to independently adjust the alignment. In addition, the alignment will not drift due to the movement of the articulated arm during the surgical procedure.

An elastomeric ring may be interposed between the coupler and the end of the endoscope so that registration is independent of the accuracy of the fabrication of the entrance configuration of the endoscope and relies entirely on the interaction between the tube and inner surface of the bore.

A two element telescope is provided in the preferred coupler. The focal powers and positions of the elements are selected so that the diameter of the beam at the entrance of the endoscope is less than the diameter of the bore. In addition, the focal plane of the beam is located just beyond the distal end of the endoscope. By using this approach, the clipping problems of the prior art are avoided and more beam power will be transmitted down the endoscope.

Further objects and advantages will become apparent from the following detailed description taken in conjunction with the drawings in which:

### Brief Description of the Drawings

Figure 1 is a cross sectional view of a conventional endoscope found in the prior art.

Figure 2 is a schematic illustration of a coupler lens and an endoscope found in the prior art.

Figure 3 is a crass sectional view of a coupler formed in accordance with the subject invention shown in conjunction with an endoscope.

Figure 4 is an exploded perspective view of the adapter flange of the coupler illustrated in Figure 3.

Figure 5 is a schematic illustration of an alternative embodiment of the coupler of the subject invention shown with an endoscope having a conical female end.

Figure 6 is a perspective view of the coupler shown in Figure 5.

### Detailed Description of the Preferred embodiments

Figure 3 is cross sectional view of a coupler 70 formed in accordance with the subject invention that has been fabricated and tested. The main body 72 of the coupler is configured to house and support the positive and negative lenses 74 and 76. Each lens is mounted in a lens cell 78 and 80. Each lens is mounted by inserting the associated lens cell into the body 72. Set screws 82 are used to lock the cells in place and permit some lateral alignment of the lenses.

Both of the lenses 74 and 76 are formed from zinc selenide which is transmissive to both visible and CO₂ radiation. The first lens 74 is provided with a focal length of 75mm. The second lens is provided with a focal length of -50mm and is spaced from the positive lens a distance of 30mm. The negative lens is also spaced from the entrance end of the endoscope 94 by 50mm. The endoscope 94 is 350mm long.

The diameter of the incoming beam from the articulated arm is on the order of 8mm. This diameter is reduced to about 4.8mm in the plane of lens 76. The diameter of the beam at the entrance to the endoscope is on the order of 4.28mm which is significantly less than the 5mm of the bore 96.

It is desirable to allow some margin of error between the diameter of the aligned beam and the inner diameter of the bore since the position of the beam entering the coupler can vary depending on the extent of angular and positional errors (run-out) induced by the movement of the articulated arm. Good design of the articulated arm can minimize run-out errors. Existing arms manufactured by the assignee herein are specified to have a positional run-out limited to 2mm and an angular run-out limited to 3 milliradians. It is anticipated that the beam entering the coupler should not exceed the limits of a 10mm error circle. In this case, the subject lens system should allow nearly all of the beam to be injected into the bore of the endoscope.

The lens system will create a focal plane at the distal end of the endoscope approximately 350mm away from the negative lens 76. The diameter of the beam at this location will be approximately 1.5mm.

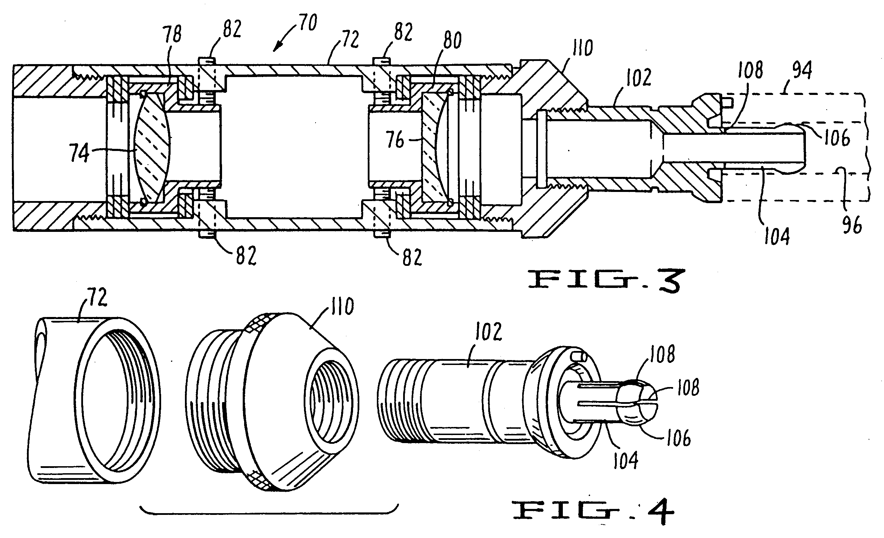

Figures 3 and 4 also illustrate the flange 102 and alignment tube 104 of the coupler 70. As can be seen, tube 104 extends only a short distance, approximately 50mm, down the length of the 350mm bore of the endoscope. This configuration should be easier to fabricate and assemble. This configuration is also designed to be used with a variety of slightly different size bores.

To achieve this goal, the end of the tube 104 is provided with a radially projecting annulus 106. The diameter of the annulus 106 can be slightly larger than the inner diameter of the bore 96 of the endoscope. The annulus 106 is provided with a plurality of axially extending slots 108. Each slot 108 is about 0.508 cm (0.200 inches) long and 0.0635 cm (0.025 inches) wide. The slots 108 allow for some compression of the tube into bores of slightly smaller diameter. This compression fit should enhance stability and help maintain alignment. A coupler nut 110 is provided to connect the flange 102 to the body 72.

As noted above there is a significant lack of accuracy in the manufacture of the prior art endoscopes. It has also been found that the axis of entrance configuration of an endoscope is often not collinear with the axis of the bore of the endoscope. Accordingly, if the flange of the coupler is rigidly connected and aligned with the axis of the entrance end of the endoscope, the accuracy of the alignment of the beam down the endoscope bore can be compromised.

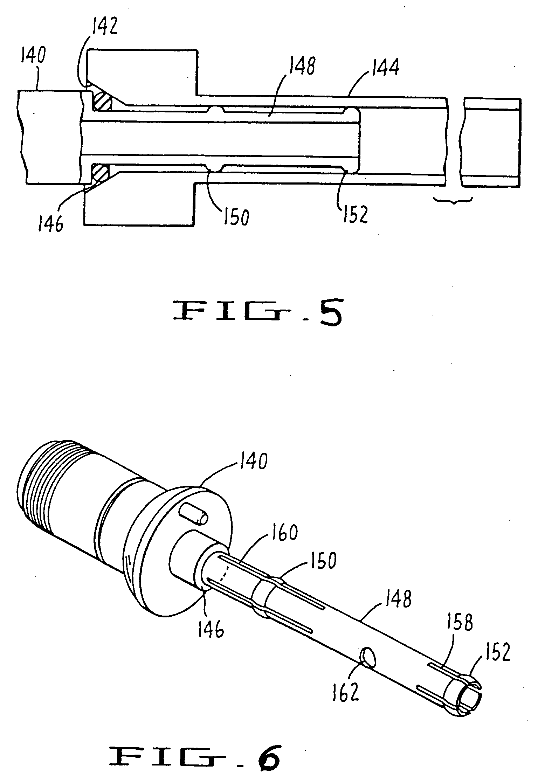

The embodiment shown in Figure 5 is designed to overcome the latter problem. In this embodiment, the flange 140 is not rigidly connected to the entrance 142 of the endoscope 144. In contrast, an elastomeric ring member 146 is inserted between the flange and the entrance 142. The ring 146 provides a seat and seal for the coupler while allowing the angle of the coupler to vary with respect to the endoscope bore. In this manner, the alignment of the coupler is not dependent on the accuracy of the construction of the entrance end configuration of the endoscope.

In this embodiment, the alignment of the coupler is based solely on the interface between the alignment tube 148 and the inner surface of the endoscope. In the embodiment shown in Figures 5 and 6, the alignment tube is provided with a pair of axially spaced annuli 150 and 152. These annuli are similar in function and structure to the annulus 106 illustrated in Figures 3 and 4. The second annulus 150 provides the extra kinematic support necessary in this configuration where the flange is floating with respect to the entrance of the endoscope.

Figure 6 is a perspective view of a coupler which has been fabricated and tested. As in the embodiment of Figures 3 and 4, the annulus 152 is provided with a plurality of axially extending slots 158 running from the end of the tube 148 a distance of about 0.76 cm (0.30 inches). A second set of slots 160 extend through the second annulus 150 and along the tube on either side of the annulus. The total length of slots 160 is on the order of 2 cm (0.80 inches). The alignment tube 148 is 5.423 cm (2.135 inches) in length.

The elastomeric member 146 would be located at the end of the flange as shown in phantom line in Figure 6. A suitable elastomeric member would be a rubber ring having a cross sectional diameter of 0.76 to 1.02 cm (0.3 to 0.4 inches) and formed from silicone rubber.

A purge gas is typically supplied to the treatment site through a fitting on the side of the endoscope (not shown). Hole 162 is provided in tube 148 to facilitate the flow of the purge gas.

The concept of using an elastomeric member as an interface between the flange and the endoscope can be extended to mating configurations other than the one shown in Figure 5. For example, the ring could be used where the entrance end of the endoscope has a male conical configuration. A ring and flange structure could also be designed for use with the planar mating surface shown in Figure 3.

In summary, there has been disclosed a new coupler for use with medical lasers. The coupler includes an axially extending tube receivable in the bore of an endoscope to improve alignment. In addition, a telescope optical system is disclosed for reducing the clipping of the beam at the entrance to the endoscope.

## Claims

1. A coupler for coupling a medical laser beam delivery system to an elongate instrument, the delivery system including an arm for delivering a laser beam, and said instrument (94) having an axially extending bore (96) through which the laser beam is transmitted, the coupler having a body (72) with one end adapted to be connected to the said arm and the other end including an adapter flange (102) having an axially projecting alignment tube (104) receivable within the bore of the instrument (94), characterised in that the adapter flange (102) is configured or adapted to mate with the entrance of the instrument (94), and in that the alignment tube (104) includes a radially projecting annulus (106) having an outer diameter substantially corresponding to the inner diameter of the bore of the instrument (94), and in that the annulus (106) includes at least one axial slot (108) to permit some compression of the annulus (106).

2. A coupler according to claim 1, characterised in that the alignment tube (46) is not sufficiently long to extend along the entire length of the bore of the instrument (42).

3. A coupler according to claim 1, characterised by an elastomeric member (146) disposed to be located between said flange (140) and the entrance to the instrument to provide a seat for the flange without substantially limiting the alignment of the coupler.

4. A coupler according to claim 1, characterised in that the alignment tube (148) includes a pair of axially spaced, radially projecting annuli (150,152) each having an outer diameter substantially corresponding to the inner diameter of the bore of the instrument.

5. A coupler according to claim 4, characterised in that each said annulus (150,152) includes at least one axial slot (158,160) providing flexibility and allowing some variation in the outer diameter of the annulus.

6. A coupler according to any preceding claim, characterised in that a first focusing element (74) is located in said body; and
a second focusing element (76) is located in said body between said first focusing element and said other end, with the focal powers of said focusing elements being selected such that, in use, the diameter of the laser beam at the entrance to the instrument is less than the inner diameter of the bore of the instrument and with the laser beam being brought to a focus proximate to the opposed end of the instrument.

7. A coupler according to claim 6, characterised in that the focusing elements are lenses (50,52).

## Patentansprüche

1. Koppler zum Ankoppeln eines medizinischen Laserstrahl-Zuführsystems an ein längliches Instrument, mit folgenden Merkmalen:
das Zuführsystem umfaßt einen Arm zur Abgabe eines Laserstrahls;
das Instrument (94) weist eine axial sich erstreckende Bohrung (96) auf, durch die der Laserstrahl übertragen wird;
der Koppler weist ein Gehäuse (72) auf, dessen eines Ende zur Verbindung mit dem Arm ausgebildet ist und dessen anderes Ende einen Adapterflansch (102) mit einem axial vorstehenden Ausrichterohr (104) umfaßt, welches innerhalb der Bohrung des Instruments (94) aufnehmbar ist,
dadurch gekennzeichnet, daß der Adapterflansch (102) so konfiguriert oder angepaßt ist, daß er zu der Einführungsstelle des Instruments (94) paßt,
daß das Ausrichterohr (104) einen radial vorstehenden Ring (106) umfaßt, dessen Aussendurchmesser im wesentlichen dem Innendurchmesser der Bohrung des Instruments (94) entspricht, und
daß der Ring (106) mindestens einen axialen Schlitz (108) umfaßt, um eine Zusammendrückung des Ringes (106) zu ermöglichen.

2. Koppler nach Anspruch 1,
dadurch gekennzeichnet, daß das Ausrichterohr (46) nicht genügend lang ist, sich entlang der gesamten Länge der Bohrung des Instruments (42) zu erstrecken.

3. Koppler nach Anspruch 1,
dadurch gekennzeichnet, daß ein Elastomerteil (146) zwischen dem Flansch (140) und der Eingangsstelle zu dem Instrument angeordnet ist, um einen Sitz für den Flansch zu bilden, ohne im wesentlichen die Ausrichtung des Kopplers zu begrenzen.

4. Koppler nach Anspruch 1,
dadurch gekennzeichnet, daß das Ausrichterohr (148) zwei radial vorstehende Ringe (150,152) umfaßt, die jeweils einen Aussendurchmesser besitzen, der im wesentlichen dem Innendurchmesser der Bohrung des Instruments entspricht.

5. Koppler nach Anspruch 4,
dadurch gekennzeichnet, daß jeder Ring (150,152) mindestens einen axialen Schlitz (158,160) umfaßt, der für Flexibilität sorgt und etwas Änderung in dem Aussendurchmesser des Ringes ermöglicht.

6. Koppler nach einem der vorhergehenden Ansprüche,
dadurch gekennzeichnet, daß ein erstes Fokussierelement (74) in dem Gehäuse angeordnet ist und daß ein zweites Fokussierelement (76) in dem Gehäuse zwischen dem ersten Fokussierelement und dem anderen Ende sitzt, wobei die Brennkräfte der Fokussierelemente so gewählt sind, daß während des Gebrauchs der Durchmesser des Laserstrahls am Eintritt zu dem Instrument kleiner ist als der Innendurchmesser der Bohrung des Instruments und wobei der Laserstrahl nahe des entgegengesetzten Endes des Instruments fokussiert wird.

7. Koppler nach Anspruch 6,
dadurch gekennzeichnet, daß die Fokussierelemente Linsen (50,52) darstellen.

## Revendications

1. Coupleur pour coupler un système de transmission d'un faisceau laser médical à un instrument allongé, le système de transmission comprenant un bras destiné à transmettre un faisceau laser, et ledit instrument (94) présentant une lumière (96) s'étendant axialement, par laquelle le faisceau laser est transmis, le coupleur ayant un corps (72) présentant une première extrémité destinée à être reliée audit bras et l'autre extrémité comprenant une bride d'adaptation (102) ayant un tube d'alignement (104) faisant saillie axialement pouvant être reçu dans la lumière de l'instrument (94), caractérisé en ce que la bride d'adaptation (102) est configurée ou conçue pour s'accoupler avec l'entrée de l'instrument (94), et en ce que le tube d'alignement (104) comprend une partie annulaire (106) faisant saillie radialement, ayant un diamètre extérieur qui correspond sensiblement au diamètre intérieur de la lumière de l'instrument (94), et en ce que la partie annulaire (106) présente au moins une fente axiale (108) pour permettre une certaine compression de la partie annulaire (106).

2. Coupleur selon la revendication 1, caractérisé en ce que le tube d'alignement (46) n'est pas suffisamment long pour s'étendre sur toute la longueur de la lumière de l'instrument (94).

3. Coupleur selon la revendication 1, caractérisé par un élément élastomérique (146) disposé de façon à être placé entre ladite bride (140) et l'entrée de l'instrument pour constituer un siège pour la bride sans limiter sensiblement l'alignement du coupleur.

4. Coupleur selon la revendication 1, caractérisé en ce que le tube d'alignement (48) comprend deux parties annulaires (150, 152) faisant saillie radialement, espacées axialement, ayant chacune un diamètre extérieur qui correspond sensiblement au diamètre intérieur de la lumière de l'instrument.

5. Coupleur selon la revendication 4, caractérisé en ce que chacune desdites parties annulaires (150, 152) présente au moins une fente axiale (158, 160) procurant de la flexibilité et permettant une certaine variation du diamètre extérieur de la partie annulaire.

6. Coupleur selon l'une quelconque des revendications précédentes, caractérisé en ce qu'un premier élément (74) de focalisation est placé dans ledit corps ; et
un second élément (76) de focalisation est placé dans ledit corps entre ledit premier élément de focalisation et ladite autre extrémité, les puissances focales desdits éléments de focalisation étant choisies de manière que, lors de l'utilisation, le diamètre du faisceau laser à l'entrée dans l'instrument soit inférieur au diamètre intérieur de la lumière de l'instrument, le faisceau laser étant focalisé en un point proche de l'extrémité opposée de l'instrument.

7. Coupleur selon la revendication 6, caractérisé en ce que les éléments de focalisation sont des lentilles (50, 52).
